Europäisches Patentamt

European Patent Office

Office européen des brevets

(11) Numéro de publication: **0 352 231**
**A2**

## DEMANDE DE BREVET EUROPEEN

(21) Numéro de dépôt: **89810539.0**

(22) Date de dépôt: **14.07.89**

(51) Int. Cl.⁵: **A 61 L 9/12**

(30) Priorité: **18.07.88 CH 2745/88**

(43) Date de publication de la demande:
**24.01.90 Bulletin 90/04**

(84) Etats contractants désignés:
**BE DE ES FR GB IT NL**

(71) Demandeur: **ARIEH-COURVOISIER S.A.**
**Rampe du Pont-Rouge, 6**
**CH-1213 Petit-Lancy (CH)**

(72) Inventeur: **Courvoisier, Guy**
**48A, Route des Coudres**
**CH-1298 Céligny (CH)**

**Arieh, Simon**
**28, Avenue de la Gare des Eaux-Vives**
**CH-1208 Genève (CH)**

(74) Mandataire: **Meylan, Robert Maurice et al**
**c/o BUGNION S.A. 10, route de Florissant Case Postale 375**
**CH-1211 Genève 12 - Champel (CH)**

(54) **Evaporateur pour désodorisant.**

(57) L'évaporateur est essentiellement constitué d'un corps (1) agissant par son poids sur un réservoir souple et déformable (3) de manière à faire monter le liquide déodorant à travers un passage (5) dans un réservoir secondaire (7) à partir duquel le liquide parvient à une plaquette de matière poreuse absorbante (2) par de courtes mèches capillaires (12, 13) ou par simple écoulement.

Fig.1

EP 0 352 231 A2

## Description

### Evaporateur pour désodorisant.

Evaporateur pour désodorisant comprenant un réservoir de liquide désodorisant, un support pour une plaquette de matière poreuse absorbante situé au-dessus du réservoir et des moyens pour amener le liquide à évaporer contenu dans le réservoir à la plaquette de matière poreuse absorbante.

Les évaporateurs de désodorisant les plus connus sont constitués d'une simple boîte contenant un disque de matériau poreux absorbant, genre papier buvard, et fermée par un couvercle que l'on dévisse partiellement pour libérer des ouvertures latérales. Dans un tel évaporateur, les substances les plus volatiles s'évaporent en premier lieu. L'effet du dispositif n'est donc pas constant. En outre sa durée de vie est courte et l'ensemble doit être jeté après usage.

On connaît également un évaporateur constitué d'un flacon muni d'un couvercle constitué en partie d'un disque en matière poreuse absorbante et muni d'une mèche en cellulose ou en acétate de cellulose plongeant dans le liquide contenu dans le flacon. ce dispositif est donc rechargeable et il peut avoir une capacité importante. Toutefois, certains composants du liquide désodorant ont tendance à détériorer les propriétés capillaires de la mèche, de telle sorte que si l'évaporateur fonctionne bien lorsqu'il est plein, il fonctionne beaucoup moins bien lorsqu'il est à moitié vide et il ne peut être rechargé valablement qu'un nombre limité de fois.

On connaît également une exécution plus sophistiquée comprenant également un flacon muni d'un couvercle portant un disque en matière poreuse absorbante et une pipette permettant de faire monter une certaine quantité de liquide déodorant contenu dans le flacon au niveau du disque poreux. La recharge du disque poreux ne se fait donc pas automatiquement.

La présente invention a pour but de réaliser un évaporateur à réservoir dans lequel l'alimentation de la plaquette ou disque en matière poreuse absorbante est assurée de façon automatique, continue et constante.

L'évaporateur selon l'invention est caractérisé par le fait que le réservoir est déformable verticalement et que les moyens pour amener le liquide à évaporer du réservoir à la plaquette en matière poreuse absorbante sont constitués par le support de cette plaquette lui-même reposant librement sur le réservoir et pressant sur lui par son poids, ce support présentant un passage de communication entre le réservoir et un réservoir secondaire formé dans le support et destiné à recevoir une quantité déterminée de liquide expulsé du réservoir par le poids du support, des moyens étant prévus pour mettre en communication ledit réservoir secondaire avec la plaquette en matière poreuse absorbante.

L'évaporateur est de préférence rechargeable par le remplacement du réservoir.

Un équilibre s'établit entre la hauteur de la colonne de liquide s'élevant du réservoir et le poids du support. Le niveau du liquide dans le réservoir secondaire reste donc constant. Du réservoir secondaire à la plaquette en matière poreuse absorbante, le liquide peut être amené par divers moyens, notamment par une ou plusieurs courtes mèches capillaires ou par simple écoulement.

Le dessin annexé représente, à titre d'exemple, deux formes d'exécution de l'invention.

La figure 1 est une vue en coupe axiale d'un évaporateur selon une première forme d'exécution.

La figure 2 est une vue en coupe axiale d'un évaporateur selon une seconde forme d'exécution.

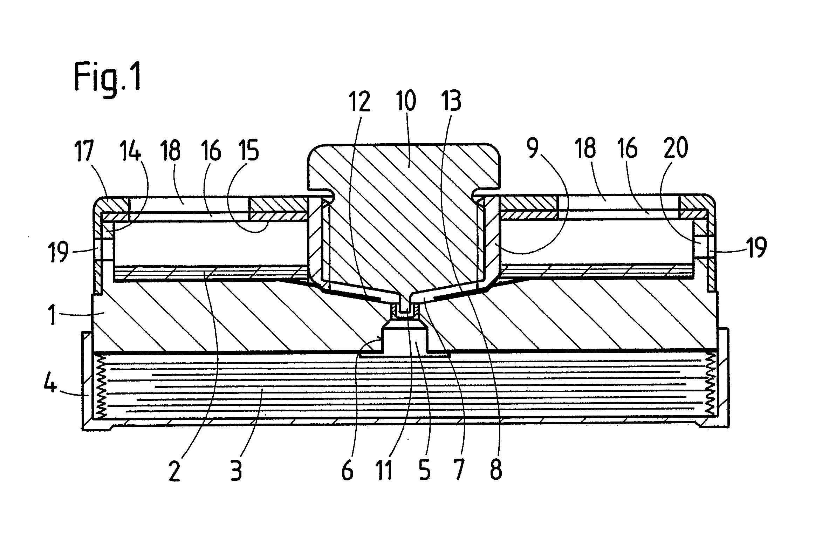

L'évaporateur représenté à la figure 1 comprend un corps cylindrique 1 servant de support pour un disque 2 en matière poreuse absorbante, par exemple du papier buvard. Le corps 1 repose librement sur un réservoir 3 déformable verticalement, réalisé de préférence en une matière mince et très souple. ce réservoir 3 peut être plissé en accordéon comme représenté sur les dessins ou constitué par une simple capsule. Ce réservoir 3 est contenu dans une cuvette cylindrique 4 dans laquelle le corps 1 coulisse librement. Le réservoir 3 est muni en son centre d'un embout 5 s'engageant dans un canal vertical 6 du corps 1, canal qui débouche dans un réservoir secondaire 7 formé dans le corps 1 par une dépression 8. En son centre, le corps 1 présente un prolongement vertical tubulaire 9 muni d'un filetage intérieur pour un bouchon vissable 10. Ce bouchon 10 présente à sa base une tige 11 venant obturer l'embout 5 en position de non utilisation. La tige 11 peut en outre être utilisée pour percer l'embout 5 lors de la première utilisation du réservoir 3. Le réservoir secondaire 7 est relié au disque absorbant 2 par de courtes mèches capillaires 12 et 13 traversant des trous obliques prévus dans le corps 1. Le corps 1 présente en outre une paroi périphérique cylindrique 14 entourant le disque 2. A l'extrémité de cette paroi et autour de la partie tubulaire 9 est soudée une plaque 15 munie de perforations 16. Le tout est surmonté d'un couvercle rotatif 17 muni de perforations 18 sur sa face supérieure et de trous latéraux 19 situés à la même hauteur que des trous 20 prévus dans la paroi 14. Selon que les trous 18 et 19 sont respectivement en face ou non des trous 16 et 20, l'évaporateur est ouvert ou fermé.

La pression exercée sur le réservoir 3 par le poids du corps 1 a pour effet d'expulser une certaine quantité de liquide du réservoir 3 et de le faire monter à travers l'embout 5 dans le réservoir secondaire 7. Le niveau du liquide atteint dans le réservoir secondaire 7 se laisse facilement calculer en fonction du poids spécifique du liquide désodorant, du poids du corps 1 et de la section du corps 1. Au moyen de ces paramètres il est donc possible de déterminer le niveau que l'on désire maintenir dans le réservoir secondaire 7. Tant qu'il y a du liquide dans le réservoir 1 et que ce dernier est déformable sans offrir de résistance notable, le niveau du liquide dans le réservoir secondaire 7 est pratiquement constant. D'autre part, les mèches capillaires 12 et 13 étant courtes, le disque évaporateur 2 est alimenté de façon continue et constante jusqu'à ce que le réservoir 3 atteigne un degré de compression tel que la résistance qu'il offre à la déformation empêche la montée du liquide dans le réservoir secondaire 7. Si le réservoir 3 est constitué d'une enveloppe très mince et très souple, il est pratiquement possible de vider ce réservoir. Lorsque le réservoir 3 est vide il suffit de l'enlever et de le remplacer par un nouveau réservoir plein.

Si, par exemple, le poids du corps 1 est de 800 mN, son diamètre est de 86 mm et le poids spécifique du liquide de 9,7 mN/cm³, le liquide s'élèvera d'environ 15 mm.

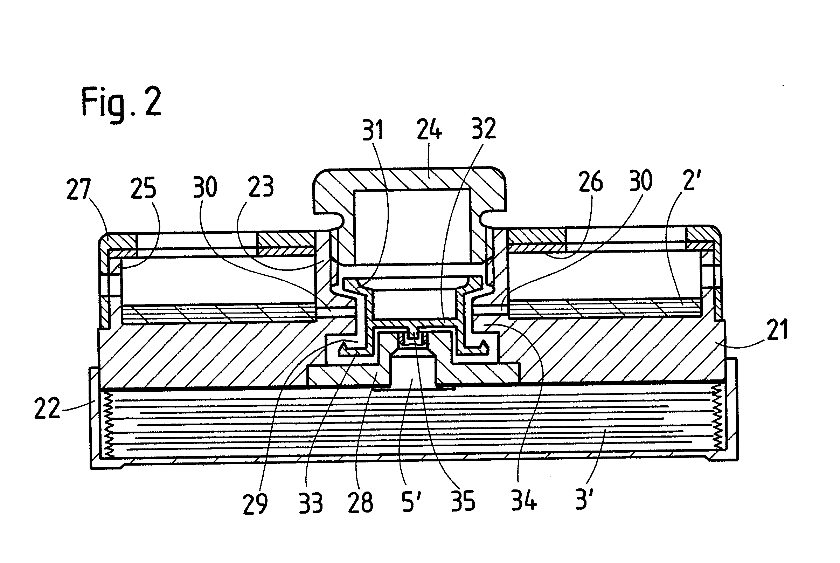

Une seconde forme d'exécution de l'épurateur selon l'invention est représentée à la figure 2. L'évaporateur comprend également un corps cylindrique 21 monté librement dans une cuvette cylindrique 22 contenant un réservoir 3' identique au réservoir 3 de la première forme d'exécution. Le corps 21 comprend, comme le corps 1, un prolongement tubulaire central 23 muni d'un bouchon vissable 24 et une paroi cylindrique périphérique 25 reliée à une plaque perforée 26 et surmontée d'un couvercle obturateur rotatif 27 muni de trous comme le couvercle 17 de la première forme d'exécution. Le corps 21 sert de support pour un disque évaporateur 2' analogue au disque 2. Le canal central ascendant est situé ici dans une pièce rapportée 28 en forme de flasque délimitant avec la partie tubulaire 23 un réservoir secondaire 29 communiquant par des trous latéraux 30 avec le disque évaporateur 2'. Les trous 30 débouchent dans l'épaisseur du disque évaporateur 2'. Les paramètres définis plus haut sont choisis de telle sorte que le liquide s'élève au moins à mi-hauteur des trous 30. Le réservoir secondaire 29 contient en outre un flotteur 31 essentiellement cylindrique, muni d'une paroi horizontale intermédiaire 32 et d'un flasque 33 à son extrémité inférieure, flasque qui s'engage sous un épaulement 34 du corps 21. La paroi intermédiaire 32 est munie en son centre d'une pointe 35 dont la fonction est analogue à celle de la tige 11 de la première forme d'exécution. Le flotteur 31 a deux fonctions. La première de ces fonctions est celle d'un clapet régulateur de débit : lorsque le liquide a atteint le niveau désiré dans le réservoir secondaire 29, par exemple un niveau situé juste au-dessus des trous 30, le flasque 33 vient s'appliquer contre l'épaulement 34 et ferme le passage du liquide vers les trous 30. Dès que le niveau du liquide a baissé par évaporation, le flotteur 30 redescend légèrement et laisse à nouveau passer le liquide. Le fonctionnement de cette forme d'exécution présente l'avantage d'être pratiquement indépendant du poids du corps 21 et de paramètres difficiles à déterminer tels que les frottements et la résistance du réservoir à la déformation. Il suffit en effet que le corps 21 présente un poids minimal. Il est dès lors possible d'utiliser un corps de poids relativement élevé pour vaincre largement les frottements et la résistance du réservoir à la déformation.

La seconde fonction du flotteur 31 est celle d'un perforateur-obturateur, fonction analogue à celle du bouchon 10 de la première forme d'exécution. Lorsqu'on visse le bouchon 24, celui-ci pousse vers le bas l'obturateur 31 et la pointe 35 de l'obturateur vient s'engager dans l'embout 5' du réservoir. A la première utilisation il perce cet embout et, ensuite, il permet de fermer cet embout.

## Revendications

1. Evaporateur pour désodorisant comprenant un réservoir de liquide désodorisant (3; 3'), un support (1; 21) pour une plaquette de matière poreuse absorbante (2; 2') situé au-dessus du réservoir et des moyens pour amener le liquide à évaporer contenu dans le réservoir à la plaquette de matière poreuse absorbante, caractérisé par le fait que le réservoir (3; 3') est déformable verticalement et que les moyens pour amener le liquide à évaporer du réservoir à la plaquette de matière absorbante sont constitués par le support de la plaquette absorbante lui-même reposant librement sur le réservoir et pressant sur lui par son poids, ce support présentant un passage de communication entre le réservoir (3; 3') et un réservoir secondaire (7; 29) formé dans le support et destiné à recevoir une quantité déterminée de liquide expulsée du réservoir par le poids du support, des moyens (12, 13; 30) étant prévus pour mettre en communication ledit réservoir secondaire avec la plaquette en matière absorbante.

2. Evaporateur selon la revendication 1, caractérisé par le fait que le réservoir secondaire (7) est relié à la plaquette de matière absorbante par au moins une mèche capillaire (12, 13) et que le support (1) est agencé de telle sorte que le niveau du liquide dans le réservoir secondaire (7) est inférieur au niveau de ladite plaquette.

3. Evaporateur selon la revendication 2, caractérisé par le fait que le réservoir secondaire (7) est fermé par un bouchon vissable (10) muni d'un organe d'obturation (11) destiné à obturer le passage de communication (6) lorsque le bouchon est vissé.

4. Evaporateur selon la revendication 1, caractérisé par le fait que ledit réservoir secondaire (29) est muni d'au moins un orifice latéral (30) mettant en communication le réservoir secondaire avec la plaquette en matière absorbante (2') et qu'il contient un obturateur à flotteur (31).

5. Evaporateur selon la revendication 4, caractérisé par le fait que le réservoir secondaire (29) est fermé par un bouchon vissable (24) susceptible d'appliquer l'obturateur à flotteur (31) contre le passage de communication entre le réservoir (3') et le réservoir secondaire (29) pour le fermer.

6. Evaporateur selon l'une des revendications 1 à 5 caractérisé par le fait que le réservoir souple (3; 3') est amovible et logé dans une cuvette cylindrique (4; 22) et que le support (1; 21) a la forme d'un piston engagé librement dans ladite cuvette.

7. Evaporateur selon l'une des revendications 1 à 6, caractérisé par le fait que le réservoir souple (3; 3') est muni d'un embout rigide (5; 5') destiné à s'emboîter dans le passage de communication entre le réservoir et le réservoir secondaire.

8. Evaporateur selon la revendication 3 ou 5, caractérisé par le fait que le réservoir souple (3; 3') est muni d'un embout rigide (5; 5') destiné à s'emboîter dans le passage de communication entre le réservoir et le réservoir secondaire, cet embout étant muni d'une membrane perforable par l'organe d'obturation (11; 35) destiné à fermer ledit passage de communication.

Fig.1

EP 0 352 231 A2

Fig. 2